(19) Europäisches Patentamt
European Patent Office
Office européen des brevets

(11) Publication number: 0 140 395 A1

(12) # EUROPEAN PATENT APPLICATION

(21) Application number: 84200882.3

(22) Date of filing: 19.06.84

(51) Int. Cl.[4]: C 07 D 233/84
A 61 K 31/415

(30) Priority: 21.06.83 IT 2170483

(43) Date of publication of application: 08.05.85 Bulletin 85/19

(84) Designated Contracting States: AT BE CH DE FR GB LI NL SE

(71) Applicant: Pietro Isnardi & C. Spa
Via XXV Aprile 69
I-18100 Oneglia Imperia(IT)

(72) Inventor: Reiner, Alberto
Via Mentana 23
I-22100 Como(IT)

(74) Representative: Dragotti, Gianfranco
c/o SAIC BREVETTI S.a.s. Via Spontini, 1
I-20131 Milano(IT)

(54) **Water soluble derivatives of 1-(2-hydroxyethyl)-2-methyl-5-nitro-imidazole having therapeutical activity, process for its preparation and related pharmaceutical compositions.**

(57) The N-(5-carboxy-5-aminopentan)-carbonic ester of 1-(2-hydroxyethyl)-2-methyl-5-nitro-imidazole is perfectly soluble in water and shows relevant trichomonacide activity.

The process for the preparation of the compound of the invention comprises reacting 1-(2-hydroxyethyl)-2-methyl-5-nitro imidazole with an alkyl- or phenyl-chlorocarbonate and hydrolizing the resulting carbonic ester, after isolation, in a water-alcohol medium with lysine hydrochloride.

The invention relates furthermore to the pharmaceutical compositions, containing the derivative according to the invention, in form suitable for the several administration routes, in combination with the usual excipients and additives.

EP 0 140 395 A1

The present invention relates to a water soluble derivative of 1-(2-hydroxyethyl)-2-methyl-5-nitro-imidazole having the following structural formula:

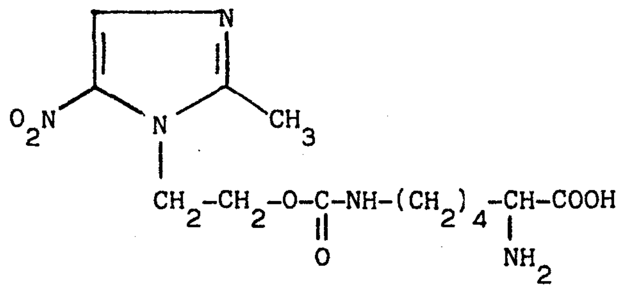

The 1-(2-hydroxyethyl)-2-methyl-5-nitro-imidazole, also referred to under the non chemical name of "metronidazole", is known and used as a drug having relevant activity in all the diseases of infectious type induced by Trichomonas vaginalis, and is used, both orally and topically, with excellent results at a posology of about 8 tablets, each containing 250 mg of active ingredient, administered in four times in only one day, thus giving a total of 2 g/day.

Owing to the insolubility in water of this active substance, the use thereof is limited, especially for the treatment of the anaerobic forms.

The main object of the present invention is that of providing a water soluble and stable form, since the drug can be thus not only used with improved efficacy, but is also useful in form of vials mainly aiming to the therapy of the anaerobic forms.

Such an object is achieved, and this is the specific gist of the present invention, through the N-(5-carboxy-5-aminopentan)-carbamic ester of 1-(2-hydroxyethyl)-2-methyl-5-nitro-imidazole, which is not only perfectly water soluble, but is also endowed with improved therapeutical properties.

According to another feature of the present invention, a process is provided for the preparation of the aforesaid water soluble derivative, comprising the steps of:

a) reacting, at a temperature of between 5 and 50°C, 1-(2-hydroxyethyl)-2-methyl-5-nitroimidazole with an alkyl or phenyl chlorocarbonate in the presence of an organic base, the corresponding carbonic ester being thus formed, and

b)hydrolizing, in water-alcohol medium and at a temperature of between 5 and 100°C, the isolated carbonic ester by means of lysing hydrochloride in the presence of triethylamine.

At the end of the hydrolysis reaction, the mixture is made acidic with hydrogen chloride and the end product is crystallized from water- alcohol solution.

In the above defined process as the alkyl chlorocarbonate, ethyl, butyl, isobutyl or phenyl chlorocarbonate is used, and the organic base is preferably selected amongst triethylamine, pyridine and piperidine, furthermore, throughout the specification, as water-alcohol solvent or mixture, a mixture of water and a lower aliphatic alcohol, preferably methanol, is meant.

The compound according to the invention is in form of a white, crystalline, ordorless, non hygroscopic powder,which is perfectly soluble in water and insoluble in anhydrous alcohols, acetone and chloroform, having melting point of 192-193°C.

Moreover the compound of the invention has shown, in the preliminary tests, greater microbiological activity, whereby a therapeutical dosage lower than that of metronidazole is made possible, the therapeutical effect being the same, and thus an improved therapeutical index is obtained.

Nevertheless for the therapeutical administration of the compound of the invention the already known dosages of metronidazole are foreseen to get improved effects.

A further object of the present invention resides in the pharmaceutical useful forms, such as capsules, tablets, normal and lyophilized ampoules, creams, ovules, washes or microtablets coated with the standard materials used for the delayed release forms and contained in only one capsule.

For the delayed release form the microtablets must be dosed according to the number and not by weight when are filled into the capsule, since this form is formulated by using part of microtablets which are immediately bioavailable, without any coating, and part of coated microtablets, which are bioavailable over about 8 to 12 hours, whereby the drug is used two times within 24 hours.

For the other pharmaceutical forms there are on the contrary used the normal excipients, vehicles etc, as well as the normal formulating techniques.

The following example illustrates, without limiting meaning, the process of the present invention.

EXAMPLE

a) 0.584 moles of 1-(2-hydroxyethyl)-2-methyl-5-nitro-imidazole are suspended in chloroform (or methylene chloride or carbon tetrachloride) in the presence of an excess of piperidine and 0.634 moles of phenyl chlorocarbonate are slowly added, at a temperature of between 5°C and 50°C.

After 12 hours reaction, the reaction mixture is discharged and the organic phase is concentrated under vacuum.

A crystalline product is obtained, which can be repeatedly crystallized from alcohols and then dried for the use in the second step, until a constant melting point of 106°C-108°C is obtained.

The process is likewise carried out using ethyl, butyl or isobutyl chlorocarbonate, with identical results.

B) In a water-alcohol solution 0.44 moles of 1-(2-hydroxyethyl)-2-methyl-5-nitro-imidazole phenyl carbonate are added and 0.60 moles of lysine hydrochloride are then supplemented by heating up to dissolution.

Then a stoichiometrically calculated amount of triethylamine is added and the mixture is maintained at a reaction temperature of between 5°C and 100°C for a time of between 3 and 25 hours.

The reaction mixture is made acidic with HCl and, after concentration, the N-(5-carboxy-5-aminopentan)-carbamic ester of 1-(2-hydroxyethyl)-2-methyl-5-nitroimidazole is isolated in a raw form.

This product is repeatedly crystallized in the presence of silica gel (granule size: 70-230 mesh) from a water-alcohol mixture, whereby a white product is obtained, with a yield of between 20% and 60%, having melting point of 192-193°C.

Like results are obtained upon identically repeating the process, except that ethyl-, butyl- and isobutyl-carbonate of 1-(2-hydroxyethyl) 2-methyl-5-nitro-imidazole is used.

In the enclosed drawings the specific characteristics of the compound of the invention are shown. More particularly:

Fig. 1 shows the U.V. spectrum;

Fig. 2 shows the I.R. spectrum;

Fig. 3 shows the N.MR. spectrum; and

Fig. 4 shows the high pressure liquid phase chromatography (HPLC) spectrum.

The compound of the invention has been pharmacologically tested.

As regards the toxicity the following results were found:

| | | |
|---|---|---|
| a) in the rat | per os | $LD_{50}$ = 1624 mg/kg |
| | i.p. | $LD_{50}$ = 870 mg/kg |
| b) in the mouse | per os | $LD_{50}$ => 4000 mg/kg |
| | i.p. | $LD_{50}$ => 4000 mg/kg |

The minimum inhibiting concentration (MIC) was investigated in five strains of Trichomonas vaginalis, in comparison with metronidazole.

Metronidazole was tested in form of a solution in dimethyl sulfoxide at a concentration of 1 mg/ml whereas the compound of the invention was tested in form of a water solution at a concentration of 1 mg/ml.

The MIC for metronidazole was on the average 5 mcg/ml whereas for the compound of the invention was 2.5 mcg/ml. Kinetic tests were carried out in the rat with the compound of the invention and it was found that it is not converted into metronidazole neither in the organism nor in acidic environnment.

More particularly it was found that the urinary excretion of the compound of the invention is of small entity after parenteral administration, it being less than 30%, which is an index of a not negligible biotransformation.

To sum up the derivative of the present invention does not only fulfill the above mentioned purposes, but is also endowed with a therapeutical activity greater than metronidazole and is not a pro-drug since no decomposition thereof in the organism takes place after the administration.

# CLAIMS

1) N-(5-carboxy-5-aminopentan)-carbonic ester of 1-(2-hydroxyethyl)-2-methyl-5-nitroimidazole having the formula:

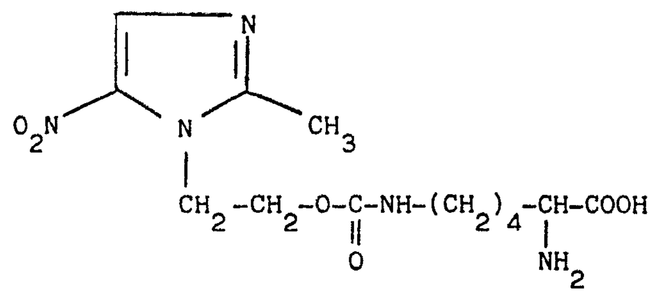

2) A process for the preparation of the ester according to claim 1, characterized by the steps of:

(a) reacting, at a temperature of between 5 and 50°C 1-(2-hydroxyethyl)-2-methyl-5-nitroimidazole with an alkyl- orphenyl-chlorocarbonate, in the presence of an organic base, giving place to the corresponding carbonic ester and

(lb) hydrolizing, in a water-alcohol medium and at a temperature of between 5 and 100°C, the carbonic ester with lysine hydrochloride in the presence of an organic amine.

3) A process according to claim 2, characterized in that said alkylchlorocarbonate is selected among ethyl-, butyl and isobutyl-chlorocarbonate.

4) A process according to claim 2, characterized in that said organic base is selected among triethylamine, pyridine and piperidine.

5) A process according to claim 2, characterized in that said carbonic ester is isolated before the hydrolysis.

6) A process according to claim 2, characterized in that said organic amine is triethylamine.

7) Pharmaceutical composition having trichomonacidal activity, characterized by containing, as the active ingredient, the ester according to claim 1, together with the usual excipients and additives.

8) Pharmaceutical composition according to claim 7, characterized by being in form suitable for oral administration.

9) Pharmaceutical composition according to claim 8, characterized by being in a delayed release form.

10) Pharmaceutical composition according to claim 7, characterized by being in

form suitable for the administration by parenteral route.



11) Pharmaceutical composition according to claim 7, characterized by being in form suitable for the administration by rectal route.

12) Pharmaceutical composition according to claim 7, characterized by being in form suitable for the administration by vaginal route.

13) Pharmaceutical composition according to claim 7, characterized by being in form suitable for the administration ty topical route.

Fig.1

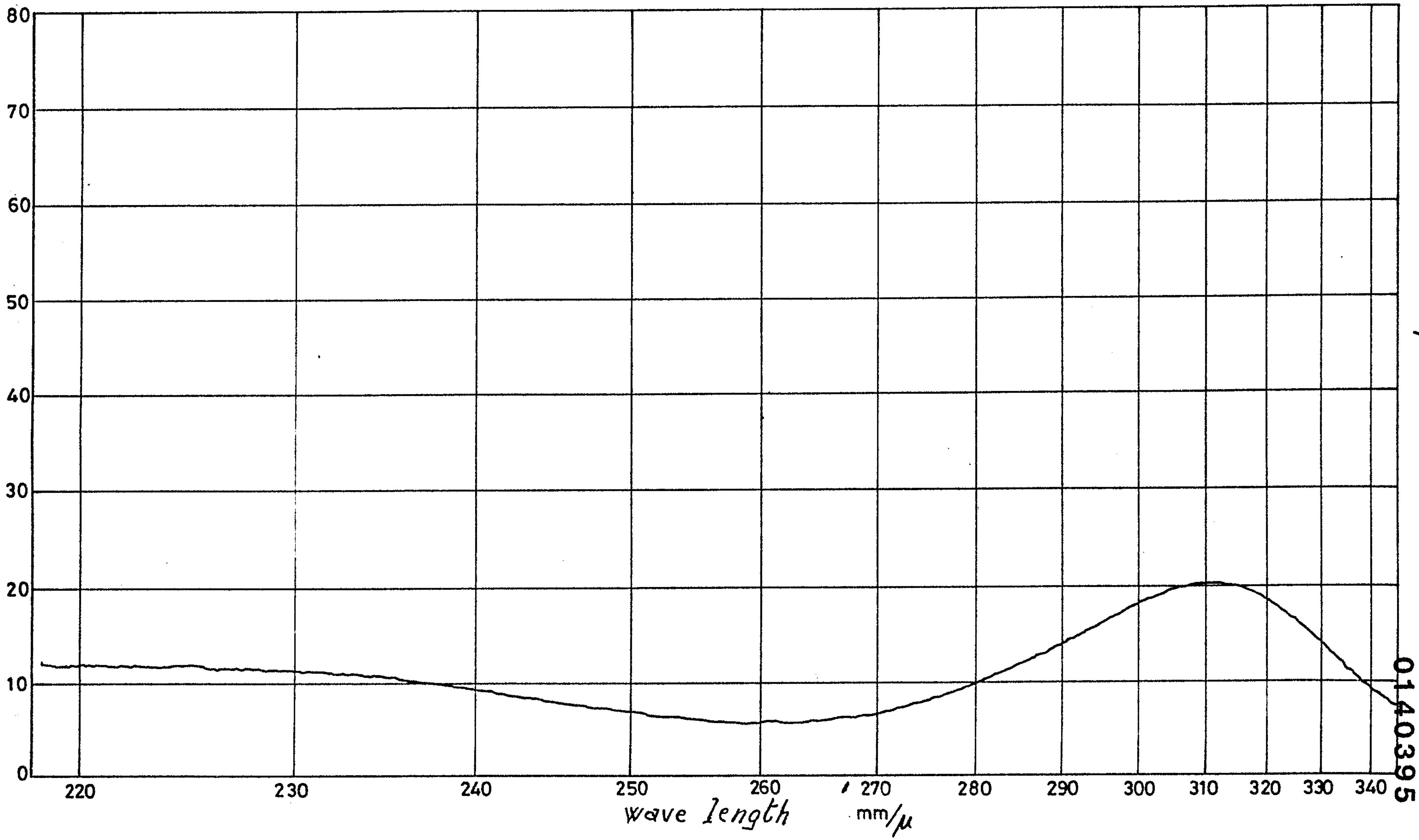

80
70
60
50
40
30
20
10
0
220
230
240
250
260
270
280
290
300
310
320
330
340
wave length mm/μ

0140395

Fig.2

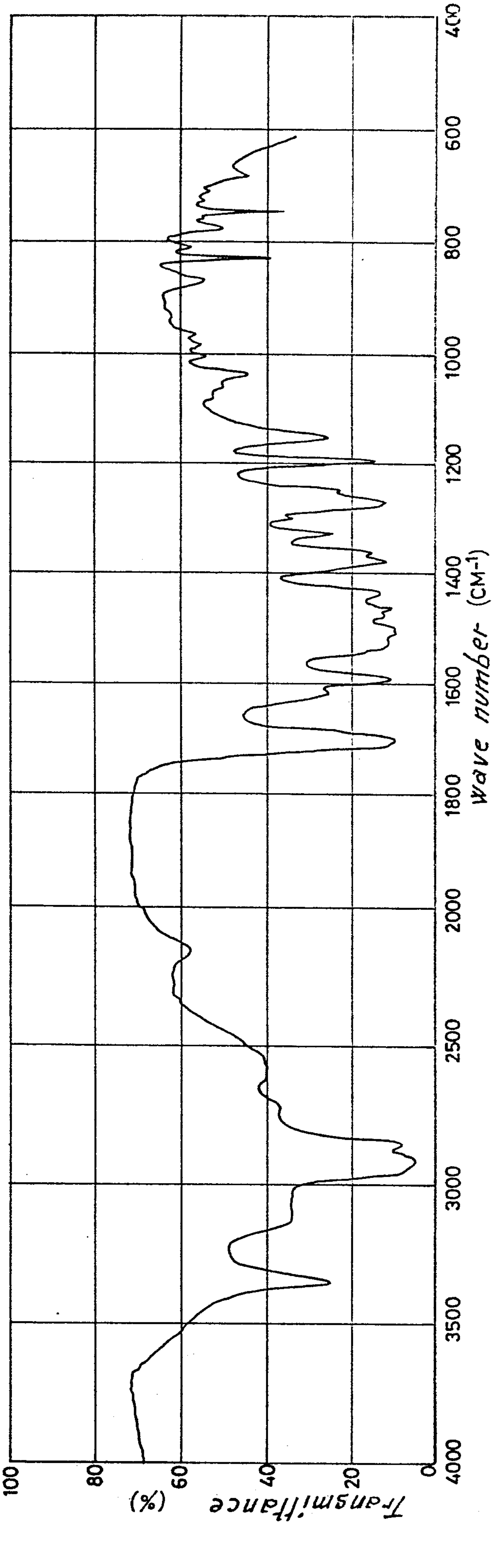

Fig.3

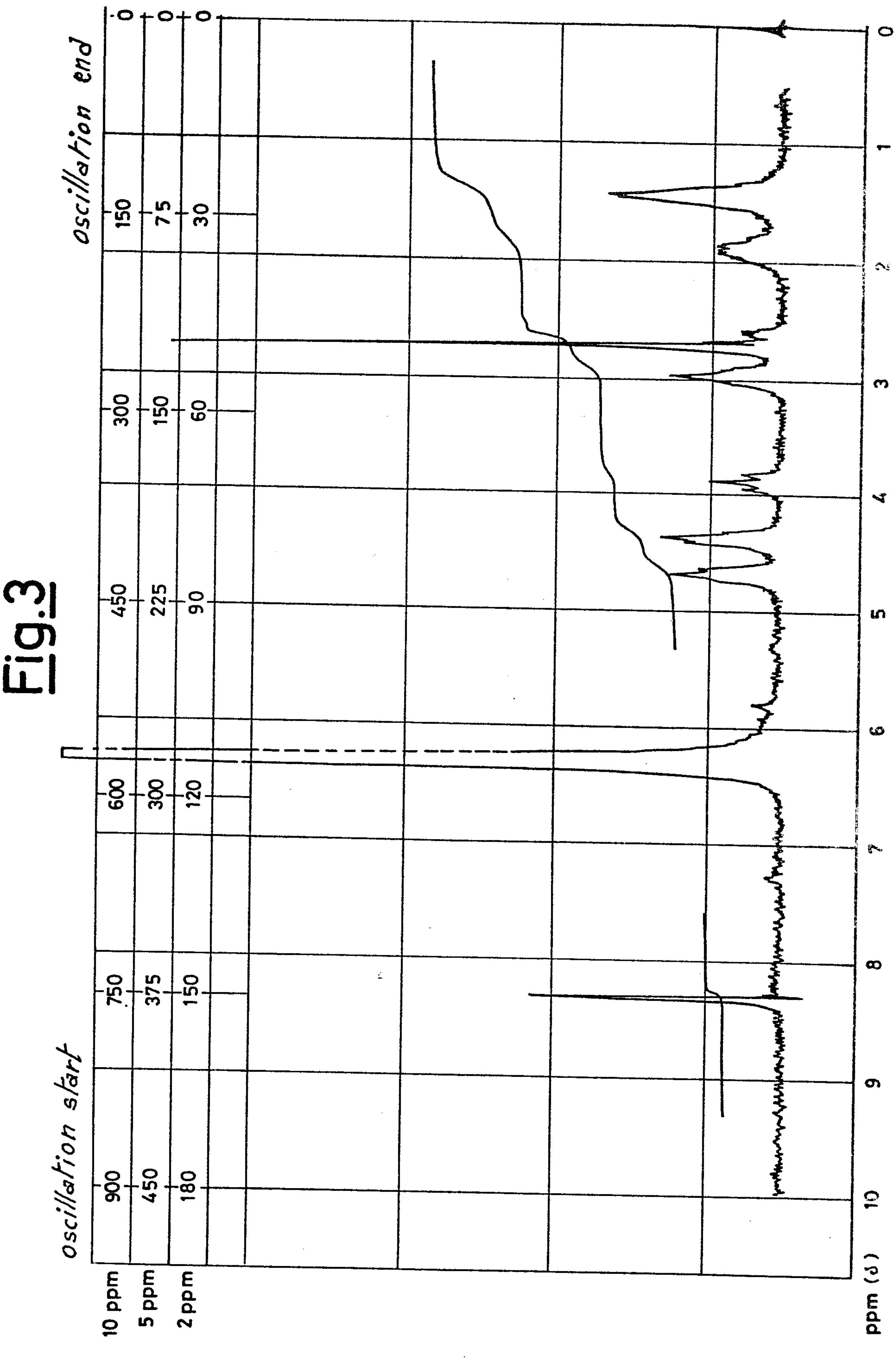
oscillation start
oscillation end
10 ppm
5 ppm
2 ppm
900 750 600 450 300 150 0
450 375 300 225 150 75 0
180 150 120 90 60 30 0
ppm (δ)
10 9 8 7 6 5 4 3 2 1 0

Fig.4

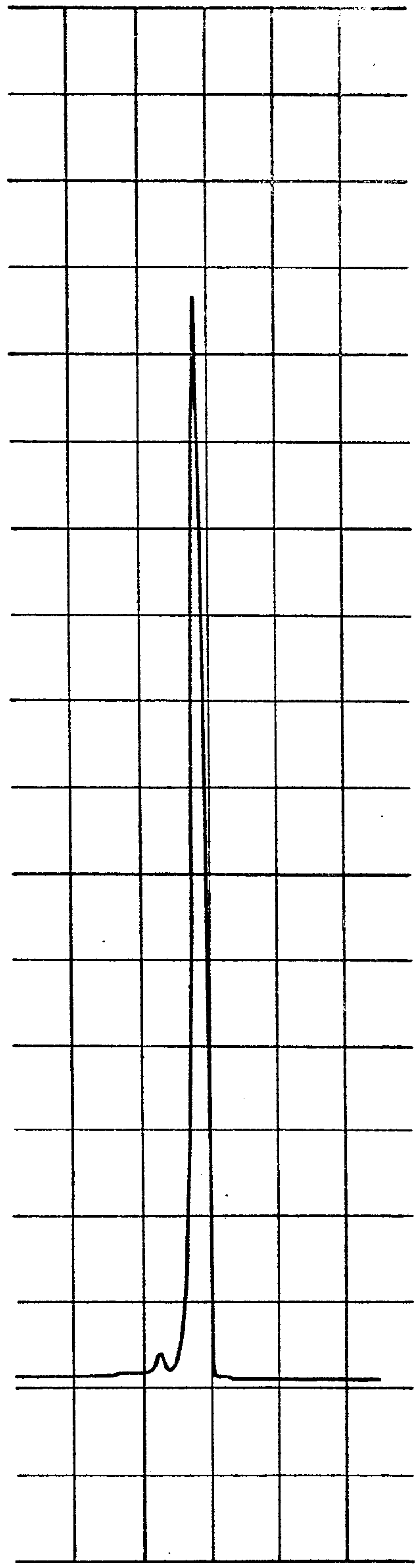

European Patent Office

# EUROPEAN SEARCH REPORT

0140395

Application number

EP 84 20 0882

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 054 504 (RHONE-POULENC)<br>--- | | C 07 D 233/94<br>A 61 K 31/415 |
| A | FR-A-2 152 382 (RHONE-POULENC)<br>--- | | |
| A | FR-A-2 234 002 (JANSSEN)<br>--- | | |
| A | GB-A-2 089 208 (LAB. SILANES)<br>--- | | |
| A | FR-A-1 393 963 (RHONE-POULENC)<br>----- | | |
| | | | TECHNICAL FIELDS SEARCHED (Int. Cl.4) |
| | | | C 07 D 233/00<br>A 61 K 31/00 |

The present search report has been drawn up for all claims

| Place of search | Date of completion of the search | Examiner |
|---|---|---|
| THE HAGUE | 13-11-1984 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82